# EUROPEAN PATENT APPLICATION

(11) **EP 2 740 467 A1**
(43) Date of publication of application: **11.06.2014**
(21) Application number: 12196302.9
(22) Date of filing: 10.12.2012
(51) Int. Cl.: A61K 8/34, A61Q 9/02, A61K 8/04

(54) **Pumpable cosmetic composition with a high humectant content and foaming properties**

(71) Applicant: OTC GmbH, 46047 Oberhausen (DE)
(72) Inventor: Fiebrig, Benjamin, 46119 Oberhausen (DE)
(74) Representative: Michalski Hüttermann & Partner Patentanwälte

(57) **Abstract**

The present invention relates to a pumpable cosmetic composition with a high humectant content and foaming properties, the use of such cosmetic composition and a kit of parts containing said cosmetic composition.

## Description

### FIELD OF THE INVENTION

The present invention relates to a pumpable cosmetic composition with a high humectant content and foaming properties, the use of such cosmetic composition and a kit of parts containing said cosmetic composition.

### BACKGROUND OF THE INVENTION

The customer acceptance of cosmetic applications is mainly governed by a couple of quality characteristics among which can be identified cosmetic efficacy, safety, ease of use, environmental impact and, of course price. Inasmuch as all of these characteristics are interconnected to a certain extend it becomes apparent that it is difficult for products to address all criteria in an equal, high quality manner. This is especially true for rinse-off cosmetic skin or hair care products where the overall treatment is less defined and the inter-subject variability with respect to the application time, amount of material used and exact procedure is higher compared to leave-on cosmetics. Additional unpredictability may exist if the cosmetic formulation is target to a phase change right before the cosmetic routine. This, for instance, is the case in cosmetic protocols where a foam application is state of the art and due to environmental reasons the foam shall be generated by purely mechanical means using air without any extra propellant. Due to the fact that foam dynamic properties are until know scientifically not very OD 41688 / UAM well understood it is hardly predictable a priori, if a given composition will yield the desired characteristics.

One possible solution to provide a foamable composition which can be applied to body surfaces is described in the US 2010/0069505 A1. Here, an alcoholic foam composition is disclosed, which can be dispensed as a foam via a pump-foam system containing a) at least 52 to ≤ 99 wt % of an alcohol or mixture of alcohols, b) a surfactant or a surfactant mixture, c) at least one polyalkylene glycol, d) optionally, at least one foam stabilizer, e) optionally, at least one member selected from the group consisting of cosmetic auxiliaries, adjuvants, active ingredients, and mixtures thereof, and f) optionally water. The surface tension of component b) lies in the range of +-15 dyn/cm of the surface tension of component a) or corresponds to the surface tension of component a), and the sum of components a) to f) is 100 wt % relative to the total quantity of the foam composition. The reason for the high alcohol content is to ensure a safe use of the composition as disinfectant. This primary application purpose becomes also apparent based on the selection of the alcohol component, i.e. only mono-olic alcoholic substances (e.g. methanol, ethanol etc.) are used in concentration higher than 52 wt%. No di- or polyols are disclosed in high concentrations within the foam. This high mono-ol content is effective as a disinfectant, but disadvantageous in cosmetic applications, because it is known that such kind of alcoholic substances are able to disturb lipid barriers and result in dry skin or hair.

Another composition for a propulsion gas-free shaving foam is described in DE 10 2006 001 755 A1. Here the use of a mixture comprising surfactant 5-25 wt.% from at least an anionic or non-ionic surfactant, at least an oil or cosmetic moisturizer 0.5-15 wt.% under exclusion of mineral oil or mineral oil based moisturizers, ethanol and/or ethanolic biomass distillates 4-25 wt.%, polyol 0-25 wt.%, further additives 0-25 wt.% and water up to 100% for foaming a propulsion gas-free shaving foam is disclosed. The shaving foam can be delivered by means of a pumping donor. This shaving foam also discloses high contents of mono-ols like methanol, ethanol etc., which are required to stabilize the composition microbiologically. This is disadvantageous, because this concentration range of the alcoholic mono-ol component may disturb lipid barriers and lead to dry skin or hair.

Nevertheless, none of the above mentioned patent documents is able to disclose a foamable skin and hair care composition adding a number of special benefits to a cosmetic rinse-off treatment, i.e.
- rich and creamy foam, which can be generated by the means of a mechanical pump using air, without any artificial propellant,
- clear and transparent formulation also applicable to very sensitive skin,
- no need to use preservatives,
- protection of the skin lipid barrier and preservation of the acid pH-mantle,
- pleasant warming experience after application to the skin,
- easy to rinse off,
- improved glidant properties also for shaving applications and
- very gentle to razor blades, no corrosive effects detectable.

### SUMMARY OF THE INVENTION

The present invention has the object of providing such an improved, foamable rinse-off skin and hair care composition and to solve the shortcomings of the prior art by a composition according to claim 1. Preferred embodiments of the invention are disclosed in the additional claims.

### DETAILED DESCRIPTION OF THE PREFERRED EMBODIMENTS

This object is achieved according to the invention by a foamable, rinse-off cosmetic composition, characterized in that the composition comprises
a) > 25 weight-% and ≤ 95 weight-% of at least one low molecular weight polyol,
b) ≥0.5 weight-% and ≤ 10 weight-% of at least one foam stabilizing agent,
c) ≥ 0.5 weight-% and ≤ 15 weight-% of at least one surfactant,
d) ≥ 0 weight-% and ≤ 50 weight-% of water,
e) ≥ 0 weight-% and ≤ 10 weight-% of at least one cosmetic or therapeutic active and
f) ≥ 0 weight-% and ≤ 10 weight-% of at least one auxiliary substance,
wherein the components a) to f) add up to 100 weight-%.

It was surprising that such a cosmetic compositions can easily be dispensed in the form of durable and smooth foam even via a simple standard pump-foam system, without spontaneous foam breaking. This feature is especially astonishing, because the high polyol content is heavily influencing the viscosity and the surface tension of the system, which is one important parameter in foam stability. Without being bound by the theory it is assumed that due to their water binding capacities the high polyol content reduces the evaporation of water from the foam lamellae, thus leading to an increased foam stability. In addition, due to the fact that the polyols are known for their intense water binding capacities, this composition leads to a long lasting humidification of the skin and the hair. Furthermore, within an cosmetic skin care application a warming of the skin is detectable, which might be attributed to the reduction of the trans epidermal water loss due to the water binding capacities of the polyols on the skin surface. In certain embodiments of the invention there is no need of a further preservation of the system, because the high polyol and low water content results in a very low water activity within the composition and thus leads to a self preserving system. Especially the use as a rinse-off cosmetic composition is inventively advantageous, because based on a low oil content the composition is easily wiped-off by a moistened cloth or rinsed-off by an aqueous solution. Rinse-off composition are intended not be left on the body surface.

The cosmetic composition can be foamed by any conventional means, i.e. for instance by a manually operated pump dispenser or a pressurized can using a standard propellant like air, propane, n-butane, iso-butane, n- pentane, carbon dioxide, nitrous oxide, nitrogen or dimethylether or even chlorinated or fluorinated hydrocarbons, although the latter are less preferred due to environmental considerations.

The composition is especially suitable for a cosmetic treatment, i.e. a treatment of human body surfaces with the intention to exclusively or mainly to clean them, perfume them, change their appearance, and/or correct body odours, and/or protect them or keeping them in good condition. Nevertheless, also medicinal or medical applications are within the scope of the invention.

Surprisingly it has been found that in comparison to standard cosmetic recipes which include polyols usually in concentration ranges up to 10 %, the incorporation of at least one low molecular weight polyol a) in a concentration range between > 25 weight-% and ≤ 95 weight-% is advantageous with respect to foam smoothness, foam richness, warming effect on the skin and hydrating properties. The at least one low molecular weight polyol have a molecular weight below 500 g/mol comprises at least two carbon-atoms and at least two OH-moieties. The low molecular weight polyol may be just a single substance or a mixture of different polyols belonging to the class of low molecular weight polyols. Mono-ols like , ethanol, 1-propanol, 2-propanol, 1-butanol, isobutyl alcohol, tert-butyl alcohol, the amyl alcohols, 1-, 2-, 3-pentanol or neopentyl alcohol as well as 1-hexanol are not within the scope of the invention for the polyol component a).

The term foam stabilizing agent b) refers to a compound or additive that increases the amount or persistence of foam produced by a surfactant system. Examples for foam stabilizers that can be used in the composition are either polymeric foam stabilizers which stabilize the foam by their rheological properties or surface active foam stabilizers which interact together with the surfactant at the water/air surface. The choice and the amount of the foam stabilizer is a function of the primary surfactant and have to be adapted accordingly. Examples for suitable foam-stabilizing agents may include lactic acid esters of mono-glycerides, cationic emulsifiers, triquaternized stearic phospholipid complexes, hydroxystearamide propyltriamine salts, lactic acid mono-glycerides, glyceryl monostearate, behentrimonium chloride, Cetrimonium chloride, propylene glycol monostearate, sodium stearoyl lactylate, silicone wax, acylisethionate, acyllactylate like sodium stearoyl lactylate, calcium stearoyl lactylate, sodium isostearoyl lactylate, sodium lauroyl lactylate, sodium caproyl lactylate, sodium cocoyl lactylate, sodium behenoyl lactylate; alkanolamides such as mono- and di-ethanolamides, N-methyl- monoethanolamide, isopropanolamides of fatty acids having about 10 to 20 carbon atoms, and PPG-hydroxyethyl cocamides, alkylamineoxide with carbon chain length in the range of 10 to 20, and polyglycerol esters, like e.g. polyglyceryl monolaurate or polyglyceryl dilaurate . Specific examples of suitable compounds include cocomonoethanolamide, cocodiethanolamide, lauryl mono/or di ethanolamide, coco mono/or di isopropanolamide, lauryl mono/or diethanolamide, myristyl mono/or diethanolamide , cocoyl N-methyl-monoethanolamide, cocoylamineoxide, laurylamineoxide, myristylamineoxide, and polypropylene glycol-2-hydroxyethyl cocoamide. Particularly useful ingredients for this invention are coco mono or diethanolamide, lauryl mono/or di ethanol amide, lauryl amine oxide and coco amine oxide.

Suitable surfactants include any surfactant known to those skilled in the art as suitable for incorporation into a hair care composition. Suitable examples of surfactants may be found in the CTFA International Cosmetics Ingredient Dictionary and Handbook, "Surfactants", 10th edition (2004). The surfactant may be selected from non-ionic surfactants, anionic surfactants, cationic surfactants, amphoteric surfactants, zwitterionic surfactants or mixtures thereof. Examples for anionic and cationic surfactants are mentioned below. Suitable amphoteric surfactants may be chosen from the group comprising alkylbetaine; alkylamidobetaine, for example cocamidopropylbetain; alkylamphomono- und diacetate, for example natriumalkylamphoacetat, dinatriumalkylamphodiacetat; alkylamphomono- and amphodipropionate; aminoglycinate; sulfobetaine; aminoxide, phospholipids and/or glucamide.

Nonionic surfactants may be selected from the group comprising fatty alcohols cetyl alcohol, stearyl alcohol, and cetostearyl alcohol (consisting predominantly of cetyl and stearyl alcohols), and oleyl alcohol, Polyoxyethylene glycol alkyl ethers (Brij), Octaethylene glycol monododecyl ether, Pentaethylene glycol monododecyl ether, Polyoxypropylene glycol alkyl ethers, Glucoside alkyl ethers, Decyl glucoside, Lauryl glucoside, Octyl glucoside, Polyoxyethylene glycol octylphenol ethers, Triton X-100, Polyoxyethylene glycol alkylphenol ethers, Nonoxynol-9, Glycerol alkyl esters, Glyceryl laurate, Polyoxyethylene glycol sorbitan alkyl esters, Polysorbate, Sorbitan alkyl esters, Spans, Cocamide MEA, Cocamide DEA, Dodecyldimethylamine oxide, Block copolymers of polyethylene glycol and polypropylene glycol: Poloxamers, Polyethoxylated tallow amine (POEA).

In a preferred embodiment of the invention the surfactants can also be selected from the group of protein tensides like hydrolyzed keratin, cocodimonium hydroxypropyl hydrolyzed collagen, cocodimopnium hydroxypropyl hydrolyzed casein, cocodimonium hydroxypropyl hydrolyzed collagen, cocodimonium hydroxypropyl hydrolyzed hair keratin, cocodimonium hydroxypropyl hydrolyzed keratin, cocodimonium hydroxypropyl hydrolyzed rice protein, cocodimonium hydroxypropyl hydrolyzed soy protein, cocodimonium hydroxypropyl hydrolyzed wheat protein, hydroxypropyl arginine lauryl/myristyl ether, hydroxypropyltrimonium gelatin, hydroxypropyltrimonium hydrolyzed casein, hydroxypropyltrimonium hydrolyzed collagen, hydroxypropyltrimonium hydrolyzed conchiolin protein.

In certain situations the inventive composition as comprises a certain amount of water d). This might either be necessary, because some of the actives, surfactants or auxiliary substances might include a certain amount of water or this amount is introduced in order to dissolve a portion of these substances. This might easy the application and availability of the ingredients.

Examples of compounds having cosmetic, optionally also, for example, dermatological, and therapeutic activity e) include: anti-acne agents, antimicrobial agents, antiperspirants, astringents, deodorants, hair-removing agents, conditioning agents for the skin, skin-smoothing agents, agents for increasing the hydration of the skin, for example glycerin or urea, sun protection agents, keratolytics, radical acceptors for free radicals, antiseptic active ingredients, active ingredients for treating the signs of skin ageing and/or agents which modulate the differentiation and/or proliferation and/or pigmentation of the skin, vitamins such as vitamin C, active ingredients having a stimulating side-effect, such as alpha-hydroxy acids, .beta.-hydroxy acids, alpha-keto acids, β-keto acids, retinoids (retinol, retinal, retinic acid), anthralins (dioxyanthranol), anthranoids, peroxides (especially benzoyl peroxide), minoxidil, lithium salts, antimetabolites, vitamin D and its derivatives; catechols, flavonoids, ceramides, wheatgerm oil and vitamin E isolated therefrom, evening primrose oil, plant lecithins (e.g. soya lecithin), sphingolipids/ceramides isolated from plants, enzymes, coenzymes, enzyme inhibitors, hydrating agents, skin-calming agents, inorganic or synthetic delustering fillers, abrasive agents.

In certain cases also auxiliary substances f) might also be present in the composition if a special cosmetic or dermatologic effect has to be achieved or for instance a special pH-range of the formulation is required. Auxiliary substances in the sense of this invention may include: high molecular weight polyols, pH-adjusting agents, buffering substances, viscosity adjusting agents such as hydrocolloids including polysaccharides, homoglycans or heteroglycans, for example carragheen, pectins, tragacanth, guar gum, locust bean flour, agar-agar, gum arabic, xanthan gum, natural and modified starches, dextrans, dextrin, maltodextrins, chitosan, glucans, such as β-1,3-glucan, β-1,4-glucan, such as cellulose, mucopolysaccharides, such as especially hyaluronic acid, synthetic polymers such as cellulose ethers, polyvinyl alcohol, polyvinylpyrrolidone, synthetic cellulose derivatives, such as methylcellulose, carboxycellulose, carboxymethyl-cellulose, especially sodium carboxymethylcellulose, cellulose esters, cellulose ethers such as hydroxypropylcellulose, polyacrylic acid, polymethacrylic acid, poly(methyl methacrylate) (PMMA), polymethacrylate (PMA), polyethylene glycols, inorganic and organic acids or bases, fatty substances, such as mineral oils, such as paraffin oils or Vaseline oils, silicone oils, vegetable oils such as coconut oil, sweet almond oil, apricot oil, corn oil, jojoba oil, olive oil, avocado oil, sesame oil, palm oil, eucalyptus oil, rosemary oil, lavender oil, pine oil, thyme oil, mint oil, cardamom oil, orange-blossom oil, soybean oil, bran oil, rice oil, rapeseed oil and castor oil, animal oils or fats, such as tallow, lanolin, butyric oil, fatty-acid esters, esters of fatty alcohols such as triglycerides, and waxes with a melting point corresponding to skin temperature (animal waxes such as beeswax, carnauba wax and candelilla wax, mineral waxes, such as microcristalline waxes, and synthetic waxes, such as polyethylene waxes or silicone waxes), amino functional silicones, like e.g. trideceth-9 PG-amodimethicone (e.g. silcare silicone sea, Clariant AG), taurate polymeres, like e.g. ammonium acryloyldimethyltaurate / VP copolymer, ammonium acrylolyldimethy taurate /beheneth-25-methacrylate crosspolymer, and sodium acryloyldimethyltaurate / VP crosspolymer, as well as all oils that are suitable for cosmetic purposes (so-called cosmetic oils), such as, for example, those mentioned in the CFTA treatise entitled Cosmetic Ingredient Handbook, 1st edition, 1988. The Cosmetic, Toiletry and Fragrance Association, Inc., Washington, cosolvents, pharmaceutically and cosmetically commonly used or other colorants and pigments, in particular those that are used primarily for the color design of the composition and not for application and color design on the human body, such as those pigments and colorants as those decorative colorants, preserving agents, softening agents and/or lubricants.

In a certain aspect of the invention auxiliary substances f) may be present in the composition comprising high molecular weight polyols. The high molecular weight polyols may exhibit a molecular weight ≥ 500 g/mol and ≤ 30 000 g/mol. Such high molecular weight polyols has been shown to contribute beneficial to the foam stability and at the same time can increase the product viscosity. One example for suitable high molecular weight polyols as auxiliary substance in the sense of the invention are Polyethylenglycols (PEGs).

In another preferred embodiment the foamable cosmetic composition comprises at least one low molecular weight polyol a), wherein the at least one low molecular weight polyol a) is selected from the group comprising branched or linear C2-C6 hydrocarbons comprising 2 - 6 hydroxyl groups. Especially low molecular weight compounds with multiple hydroxyl functional groups may comprise to the foam stability, the foam richness and the beneficial skin and hair properties of the inventive formulation. Polyols with a longer hydrocarbon chain-length might interfere with the foam formation and are less suitable for skin and hair hydration properties. Examples for suitable low molecular weight polyols comprising 2 hydroxyl groups are for instance propylenglycol, butylenglycol, ethylenglycol, diethylenglycol and/or triethylenglycol. In a preferred embodiment the polyols are non-cyclic, linear and unbranched sugar-alcohols and can be selected from the group of sugar alcohols (also known as polyhydric alcohols or glycitols). For use in the inventive composition these polyols might be polyols like glycol, glycerol, erythritol, threitol, arabitol, xylitol, ribitol, mannitol, sorbitol, galactitol and iditol.

In another aspect of the invention the foamable cosmetic composition comprises a low molecular weight polyol a), wherein the low molecular weight polyol a) is glycerin. Especially the glycerin is preferred, because glycerin might help in foam stabilization and contributes to the richness of the foam. In addition glycerin in suitable in increasing the moisture content of body surfaces and is able to penetrate for instance into deeper skin layers. This might be especially helpful in rinse-off application, where the composition is removed, rinsed-off, from the body surface after a certain application time. Theses substances which are able to penetrate into the skin or hair within the application time frame might contribute to a longer lasting cosmetic effect.

Further a foamable cosmetic composition comprising a low molecular weight polyol is within the scope of the invention, wherein the low molecular weight polyol a) is present in a concentration of ≥ 50 weight-% and ≤ 95 weight-%. Surprisingly it has been found that especially the very high low molecular weight polyol content might contribute to long lasting moisturization of the body surfaces and ease the removal of the foam from the body surface after the application time. In addition the high polyol concentration in the composition may yield a high surface concentration of the polyol on the body surfaces, which might increase the penetration performance of the polyol. Especially these high concentrations may increase the body surface temperature, because the trans-epidermal water loss might be significantly reduced.

In another preferred embodiment of the invention the foamable cosmetic composition comprises a foam stabilizing agent b), wherein the foam stabilizing agent b) is selected from the group comprising lactic acid esters of mono-glycerides, triquaternized stearic phospholipid complexes, hydroxystearamide propyltriamine salts, lactic acid mono-glycerides, glyceryl monostearate, behentrimonium chloride, Cetrimonium chloride, propylene glycol monostearate, sodium stearoyl lactylate, silicone wax. Especially the non-polymeric foam stabilizing agents might be preferred, because they are able to increase foam duration and stability without influencing the viscosity of the composition too much. This is often the case, when polymeric foam stabilizers are employed.

In an additional embodiment according to the invention the foamable cosmetic composition comprises at least a foam stabilizing agent b), wherein the foam stabilizing agent b) comprises at least palmitoyl hydrolyzed wheat protein. The palmitoyl hydrolyzed wheat protein is especially preferred as a foam stabilizing agent b), because this agent can completely be sourced from re-newable resources, is very skin friendly and the foam boosting efficacy is over a large concentration range not influenced by the low molecular weight polyol content.

Furthermore, a foamable cosmetic composition comprising at least one surfactant c) is within the scope of the invention, wherein the at least one surfactant c) is selected from the group comprising anionic or cationic surfactants. The surfactant is preferably selected from ionic surfactants. Surprisingly, it has been found, that especially the anionic or cationic surfactants might lead to high foam volumes and long foam duration. Anionic surfactant can be selected from the group comprising sulfate, sulfonate, phosphate, carboxylates, alkyl sulfates, ammonium lauryl sulfate, sodium lauryl sulfate (SDS), alkyl-ether sulfates, sodium laureth sulfate (SLES), sodium myreth sulfate, Docusates, dioctyl sodium sulfosuccinate, perfluorooctanesulfonate (PFOS), perfluorobutanesulfonate, linear alkylbenzene sulfonates (LABs), alkyl-aryl ether phosphates, alkyl ether phosphate alkyl carboxylates (soaps), sodium stearate, sodium lauroyl sarcosinate, fluorosurfactants, perfluorononanoate, perfluorooctanoate (PFOA or PFO). sulfate, sulfonate, phosphate, carboxylates, alkyl sulfates, ammonium lauryl sulfate, sodium lauryl sulfate (SDS), alkyl-ether sulfates, sodium laureth sulfate (SLES), sodium myreth sulfate, Docusates, dioctyl sodium sulfosuccinate, perfluorooctanesulfonate (PFOS), perfluorobutanesulfonate, linear alkylbenzene sulfonates (LABs), alkyl-aryl ether phosphates, alkyl ether phosphate alkyl carboxylates (soaps), sodium stearate, sodium lauroyl sarcosinate, fluorosurfactants, perfluorononanoate and/or perfluorooctanoate (PFOA or PFO).

Cationic surfactants can be preferably selected from the group comprising primary, secondary or tertiary amines, betaines, octenidine dihydrochloride; Alkyltrimethylammonium salts, cetyl trimethylammonium bromide (CTAB), hexadecyl trimethyl ammonium bromide, cetyl trimethylammonium chloride (CTAC) and/or Cetylpyridinium chloride (CPC).

In an additional characteristic of the invention the foamable cosmetic composition exhibits a specific viscosity, wherein the viscosity of the foamable cosmetic composition is ≥ 10 mPas and ≤ 1000 mPas. Preferably the viscosity of the composition may be adjusted, if necessary, for instance using rheological modifiers, to the above mentioned viscosity range. This special range may be helpful in providing a composition which may easily be foamed only by a mechanical pump dispenser. In addition, the lower viscosity limit may be helpful to provide a composition with sufficient body, which when foamed is not immediately flowing from the body surface. The composition viscosity can be determined according to methods known to the skilled in the art, for instance using a Brookfield viscometer, spindle No. 2, at a temperature of 25°C and a spindle speed of 20 rpm.

In an additional inventive aspect the foamable cosmetic composition comprises surface active and foam stabilizing agents, wherein the content of the at least one foam stabilizing agent b) and the content of the at least one surfactant c) is ≥ 1 weight-% and ≤ 20 weight-%. Surprisingly at has been found that compositions comprising a total content of surface active substances like surfactants and foam stabilizing agents in the above mentioned concentration range do yield excellent foam characteristics and are very gentle to the body surfaces. The skin or the hair is not damaged due to removal of the lipid barrier by the surface active agents.

In a further embodiment the foamable cosmetic composition is transparent or translucent. Due to the high polyol content and the inventive surfactant amount the formulation can exhibit a visual clear, transparent or translucent appearance. This is especially preferred because such appearance is very attractive to the end customer.

In another preferred embodiment the foamable cosmetic composition can be used in a skin or hair care cosmetic treatment. The inventive rinse-off composition can be especially applicable within a shaving or a hair care cosmetic treatment. The achievable foam stability and the desired rinse-off characteristics of the composition ease the application and rinse-off characteristics of the formulation. Without being bound by the theory the high polyol-content might contribute to a good moisturization of the skin and the hair even within the restricted time-scale of a rinse-off application. A skincare treatment in the sense of the inventions includes the treatment of all skin areas, namely also applications which might otherwise be addressed as body-care applications.

In an additional aspect of the invention the foamable cosmetic composition can be used in a shaving treatment. Surprisingly it has been found that the inventive composition is highly suitable for the use within a shaving treatment. Without being bound by the theory it is assumed that the high polyol content leads to a thorough moisturization of the hair and thus eases the hair removal by a blade. In addition the composition might also protect the blade surface and prevents corrosive effects.

Furthermore, in one preferred aspect of the invention the composition is removed from the body surface after an application time of 0.5 - 15 minutes. This time frame is preferred for the application of the inventive composition. A longer time scale is disadvantageous because this might lead to a sticky feeling of the dried composition on the skin. A shorter timescale might be disadvantageous, because than the effective interaction of the composition with the body surface cannot be established.

In an additional characteristic of the invention the composition is aerated and applied to a body surface by the means of a manually operated pump dispenser. Surprisingly it has been found that the inventive composition is especially suitable to be aerated just by using a manually operated pump dispenser. This method is unexpectedly possible using the inventive composition, although usually the manual operation leads to less stable and un-reproducible foams. Without being bound by the theory this method is possible due to the inventive polyol, surfactant and foam stabilizer agent concentration range. This application can be preferable, because it reduces the packaging and production costs and is environmentally friendly.

It is further within the scope of the invention to provide a kit of parts for a skin or hair care cosmetic treatment comprising a) the inventive foamable cosmetic composition, b) optionally a after-shave lotion and/or c) optionally a razor and/or d) optionally at least one applicator selected from the group consisting of a spatula, a glove, a syringe, a brush, and a comb.

### Examples:

### Example 1: Shaving foam composition

| **INCI name** | [%wt] |
|---|---|
| Sodium Cocoyl Glycinate | 12 |
| Potassium Palmitoyl Hydrolyzed Wheat Protein | 3 |
| Glycerol | 50 |
| Water | 35 |
| | |
| total | 100 |

### Example 2: Shaving foam composition

| **INCI name** | [%wt] |
|---|---|
| Sodium Lauroy Lactylate | 1.5 |
| Sodium Cocoyl Glytamate | 15 |
| Sodium Cocoyl Isethionate | 0.8 |
| Potassium Palmitoyl Hydrolyzed Wheat Protein | 1 |
| Ammonium Acryloyldimethyltaurate/VP Copolymer | 0.3 |
| Glycerol | 55 |
| Water | 26.4 |
| | |
| total | 100 |

### Example 3: 3-in-1 rinse-off hair-care composition

| **INCI name** | **[%wt]** |
|---|---|
| Sodium Cocoyl Glycinate | 7 |
| Sodium Lauroy Lactylate | 3 |
| Sodium Cocoyl Glytamate | 1 |
| Sodium Cocoyl Isethionate | 1.5 |
| Trideceth-9 PG-Amodimethicone and Trideceth-12 | 1 |
| Hydrolyzed Keratin | 1 |
| Polyquaternium-7 | 0.2 |
| Ammonium Acryloyldimethyltaurate/VP Copolymer | 0.5 |
| Glycerol | 20 |
| Water | 64.8 |
| | |
| total | 100 |

### Example 4: Rinse-off shower composition

| INCI name | **[%wt**] |
|---|---|
| Sodium Cocoyl Glycinate | 10 |
| Sodium Lauroy Lactylate | 1.5 |
| Sodium Cocoyl Glytamate | 0.5 |
| Sodium Cocoyl Isethionate | 0.8 |
| Glycerol | 25 |
| Water | 62.2 |
| | |
| total | 100 |

## Claims

1. A foamable, rinse-off cosmetic composition, **characterized in that** the composition comprises
a) > 25 weight-% and ≤ 95 weight-% of at least one low molecular weight polyol,
b) ≥ 0.5 weight-% and ≤ 10 weight-% of at least one foam stabilizing agent,
c) ≥ 0.5 weight-% and ≤ 15 weight-% of at least one surfactant,
d) ≥ 0 weight-% and ≤ 50 weight-% of water,
e) ≥ 0 weight-% and ≤ 10 weight-% of at least one cosmetic or therapeutic active and
f) ≥ 0 weight-% and ≤ 10 weight-% of at least one auxiliary substance,
wherein the components a) to f) add up to 100 weight-%.

2. The foamable cosmetic composition according to claim 1, wherein the at least one low molecular weight polyol a) is selected from the group comprising branched or linear C2-C6 hydrocarbons comprising 2 - 6 hydroxyl groups.

3. The foamable cosmetic composition according to any of the preceding claims, wherein the low molecular weight polyol a) is glycerin.

4. The foamable cosmetic composition according to any of the preceding claims, wherein the low molecular weight polyol a) is present in a concentration of ≥ 50 weight-% and ≤ 95 weight-%.

5. The foamable cosmetic composition according to any of the preceding claims, wherein the foam stabilizing agent b) is selected from the group comprising lactic acid esters of mono-glycerides, triquaternized stearic phospholipid complexes, hydroxystearamide propyltriamine salts, lactic acid mono-glycerides, glyceryl monostearate, behentrimonium chloride, Cetrimonium chloride, propylene glycol monostearate, sodium stearoyl lactylate and silicone wax.

6. The foamable cosmetic composition according to any of the preceding claims, wherein the foam stabilizing agent b) comprises at least palmitoyl hydrolyzed wheat protein.

7. The foamable cosmetic composition according to any of the preceding claims, wherein the at least one surfactant c) is selected from the group comprising anionic or cationic surfactants.

8. The foamable cosmetic composition according to any of the preceding claims, wherein the viscosity of the foamable cosmetic composition is ≥ 10 mPas and ≤ 1000 mPas.

9. The foamable cosmetic composition according to any of the preceding claims, wherein the content of the at least one foam stabilizing agent b) and the content of the at least one surfactant c) is ≥ 1 weight-% and ≤ 20 weight-%.

10. The foamable cosmetic composition according to any of the preceding claims, wherein the cosmetic composition is transparent or translucent.

11. Use of the foamable cosmetic composition according to any of the claims 1 - 10 in a skin or hair care cosmetic treatment.

12. The use according to claim 11, wherein the cosmetic treatment is a shaving treatment.

13. The use according to claim 11 or 12, wherein the composition is removed from the body surface after an application time of 0.5 - 15 minutes.

14. The use according to claim 11 - 13, wherein the composition is aerated and applied to a body surface by the means of a manually operated pump dispenser.

15. Kit of Parts for a skin or hair care cosmetic treatment comprising
a) the foamable cosmetic composition as claimed in any of the claims 1 - 10 and
b) optionally a after-shave lotion and/or
c) optionally a razor and/or
d) optionally at least one applicator selected from the group consisting of a spatula, a glove, a syringe, a brush, and a comb.
